# EUROPÄISCHE PATENTSCHRIFT

(11) **EP 1 163 885 B1**
(45) Veröffentlichungstag und Bekanntmachung des Hinweises auf die Patenterteilung: **07.03.2007**
(21) Anmeldenummer: 01108309.4
(22) Anmeldetag: 02.04.2001
(51) Int. Cl.: A61B 18/14

(54) **Endoskopisches Instrument mit zwei Elektroden**
Endoscopic instrument with two electrodes
Instrument endoscopique avec deux électrodes

(30) Priorität: 16.06.2000 DE 10028959
(43) Veröffentlichungstag der Anmeldung: 19.12.2001
(73) Patentinhaber: OLYMPUS WINTER & IBE GMBH, 22045 Hamburg (DE)
(72) Erfinder: Brommersma, Pieter, 22941 Bargteheide (DE)
(74) Vertreter: Emmel, Thomas

(56) Entgegenhaltungen:
- WO-A-97/24993
- DE-A- 4 323 585
- DE-A- 19 734 506
- US-A- 3 920 021
- US-A- 3 987 795
- US-A- 4 682 596

## Beschreibung

Die Erfindung betrifft ein endoskopisches Instrument der im Oberbegriff des Anspruches 1 genannten Art.

Endoskopische Instrumente werden durch das Schaftrohr eines in den Körper zu einem Körperhohlraum eingeführten Endoskopes mit ihrem langgestreckten Einführungsteil vorgeschoben, bis sie mit ihrem distalen Endbereich am Arbeitsort im Körperhohlraum sind. Dort können die Elektroden unter HF-Beaufschlagung zur Koagulation oder Abtragung von Gewebe verwendet werden. Typische Einsatzgebiete sind die Blase, die Prostata oder auch der Uterus.

Bei älterer monopolarer Arbeitsweise wies das Instrument nur eine Elektrode auf, von der bei Gewebekontakt der Strom durch das Gewebe nach außen zur Körperoberfläche und zu einer dort angeordneten Neutralelektrode floß. Neuere bipolare Instrumente weisen beide Elektroden im distalen Endbereich auf, zwischen denen der Strom auf kurzem Wege, also nicht durch den gesamten Körper fließt.

Bei einem Gerät gemäß DE 38 14 967 A1 sind beide Elektroden auf der Oberfläche eines Isolators benachbart zu gemeinsamem Gewebekontakt angeordnet. Diese Anordnung hat schwerwiegende Nachteile, da von den beiden in Gewebekontakt stehenden Elektroden stets nur eine ordnungsgemäß arbeiten kann.

Bei einem Instrument gemäß WO 97/24993, Fig. 9 ist eine Elektrode in bezug auf die Achse des Instrumentes seitlich zum Gewebekontakt angeordnet. Die andere Elektrode ist, gegen die erste Elektrode mit einem Isolator abgeschirmt, auf der gegenüberliegenden Seite angeordnet und steht dort in Kontakt mit leitfähiger, den Körperhohlraum füllender Flüssigkeit. Von der in Gewebekontakt stehenden aktiven Elektrode fließt der Strom über kurze Strecken durch das Gewebe, tritt dann in die Flüssigkeit aus und verläuft durch diese zur abgewandten Neutralelektrode. Der Isolator zwischen den Elektroden verhindert direkten Stromfluß durch die Flüssigkeit zwischen den Elektroden, durch den viel Generatorleistung nutzlos verbraucht würde. Das Einsatzgebiet dieses Instrumentes mit seitlich angeordneter aktiver Elektrode ist jedoch naturgemäß auf seitliches Arbeiten beschränkt. Man kann mit einem solchen Gerät in einem Körperhohlraum nur seitlich des Instrumentes gelegene Oberflächenbereiche bearbeiten. Vor dem Instrument gelegene Wandbereiche des Hohlraumes sind damit aus geometrischen Gründen nicht erreichbar.

Die nicht gattungsgemäße US-A-3 987 795 zeigt ein Instrument, das am distalen Endbereich eines langgestreckten Einführungsteiles zwei Elektroden aufweist, zwischen denen ein Isolator angeordnet ist. Dabei sind beide Elektroden in Bezug auf die Achse des Einführungsteiles seitlich angeordnet. Der Isolator ist als Körper mit ebenen Seitenflächen und einer senkrecht zur Achse des Einführungsteiles stehenden ebenen, distalen Stirnfläche ausgebildet. Die Elektroden sind an den Seitenflächen angeordnet, jedoch nicht an der Stirnfläche.

Die DE 197 34 506 A1 zeigt ein Instrument mit mehreren, auf einem verrundeten Kopf flächig angeordneten Elektroden, die durch Isolierstreifen gegeneinander getrennt sind. Diese Elektroden können einzeln oder parallel zu mehreren an denselben Pol einer HF-Quelle angeschlossen werden. Es läßt sich damit die Strombelastung verringern, indem jeweils nur die in Körperkontakt stehenden Elektrodenflächen strombeaufschlagt werden. Bipolare Beaufschlagung der Elekroden, die in der Regel zu mehreren in Körperkontakt stehen, ist nicht vorgesehen und würde die Nachteile der DE 38 14 967 A1 ergeben.

In der DE 43 23 585 A1, die die Merkmale des Oberbegriffs des Anspruchs 1 offenbart, ist ein Instrument beschrieben, das an einem verrundeten Kopf eine überwiegend seitlich angeordnete Neutralelektrode und eine zentral angeordnete, nach distal weisende Arbeitselektrode aufweist. Die Arbeitselektrode kann als Schneidnadel oder auch als verrundete flächige Koagulationselektrode ausgebildet sein. In letzterem Falle bilden beide Elektroden gemäß Spalte 6, Zeile 41 eine durchgehende, etwa halbkugelförmige Koagulationsfläche, bei der beide Elektroden in Körperkontakt stehen. Auch im Falle der distalen Nadelelektrode läßt sich angesichts der verrundeten Ausbildung der Neutralelektrode ein gleichzeitiger Kontakt beider Elektroden nicht vermeiden. Es bestehen also wiederum die Nachteile, die bereits zur DE 38 14 967 A1 erwähnt wurden. Es besteht nämlich keine saubere Trennung zwischen in Gewebekontakt befindlicher Aktivelektrode und in Flüssigkeitskontakt stehender Neutralelektrode. Ein weiterer Nachteil dieses Instrumentes besteht darin, daß sein aktiver Arbeitsbereich in beiden Ausführungsformen im distalen Spitzenbereich des verrundeten Kopfes liegt. Um mit diesem Instrument auch seitliches Arbeiten zu ermöglichen, ist eine mechanische Abwinkelung des Kopfes gegenüber der Längsachse seines Einführungsteiles vorgesehen, was mit erheblichem konstruktivem Aufwand verbunden ist.

Die Aufgabe der vorliegenden Erfindung besteht darin, ein gattungsgemäßes Instrument zu schaffen, das bei einfacher Konstruktion sowohl seitliches als auch distales Arbeiten ermöglicht, und zwar unter Gewährleistung einer sauberen Trennung zwischen in Körperkontakt bringbarer Arbeitselektrode und in Flüssigkeitskontakt stehender Neutralelektrode.

Diese Aufgabe wird mit den Merkmalen des Anspruches 1 gelöst.

Erfindungsgemäß sind die beiden Elektroden an vorzugsweise senkrecht zueinander abgewinkelt stehenden ebenen Flächen eines Isolatorkörpers vorgesehen, von denen die eine Fläche seitlich und die andere distal und senkrecht zur Achse des Einführungsteils steht. Die Elekroden sind auf diesen Flächen angeordnet und somit derart deutlich um die Kante zwischen den beiden Flächen herum gegeneinander geometrisch getrennt, daß sowohl direkter Stromfluß zwischen den beiden Elektroden, der nutzlos Energie verbrauchen würde, vermieden wird, als auch ein eindeutiges Anlegen nur der einen oder nur der anderen Elektrode an Körperflächen des Patienten ermöglicht wird. Gegen eine seitlich liegende Körperfläche wird die seitlich liegende Elektrode in Anlage gebracht, während die distal liegende Elektrode frei in der Spülflüssigkeit steht. Umgekehrt kann an einer querstehenden Körperfläche, z.B. an der gegenüberliegenden Wand der Blase, mit der distal liegenden Elektrode gearbeitet werden, während die seitliche Elektrode frei in der Flüssigkeit steht. Es wird daher sichergestellt, daß in jedem der beiden Anwendungsfälle immer nur die eine Elektrode als Arbeitselektrode in Gewebeanlage und die andere Elektrode als Neutralelektrode in Flüssigkeitskontakt steht. Damit werden die bei der DE 38 14 967 A1 erwähnten Nachteile vermieden und es wird ein konstruktiv sehr einfaches Instrument geschaffen, mit dem sowohl seitlich als auch distal gearbeitet werden kann, ohne daß dazu die aufwendige Schwenkkonstruktion der DE 43 23 585 A1 erforderlich wäre.

Dabei sind vorteilhaft gemäß Anspruch 2 die Elektroden flächig ausgebildet. Auf diese Weise sind sie insbesondere gut zur Koagulation bzw. zur flächigen Gewebeabtragung nach dem sogenannten Vaporisationsverfahren geeignet.

Vorteilhaft sind die Elektroden dabei gemäß Anspruch 3 allseitig von den Flächen des Isolators überragt. Bei Anlegung einer Elektrode auf die Gewebeoberfläche liegt auch der die Elektrode umgebende Rand der Isolatorfläche auf dem Gewebe, so daß sich eine besonders gute Stromabschirmung zwischen den Elektroden ergibt.

In den Zeichnungen ist die Erfindung beispielsweise und schematisch dargestellt.

Es zeigen:
- Fig. 1:: ein erfindungsgemäßes Instrument in Seitenansicht in einem Körperhohlraum in Arbeitseingriff und
- Fig. 2:: eine Stirnansicht des Instrumentes.

Das in den Figuren dargestellte Instrument weist einen geraden langgestreckten Einführungsteil 1 auf, an dessen distalem Ende ein Isolatorkörper 2 angeordnet ist, beispielsweise aus geeigneter Keramik bestehend. Der Isolatorkörper 2 bildet eine gegenüber der Achse des Einführungsteiles 1 seitlich angeordnete ebene Seitenfläche 3 und eine senkrecht zur Achse des Einführungsteiles stehende distale Stirnfläche 4 aus.

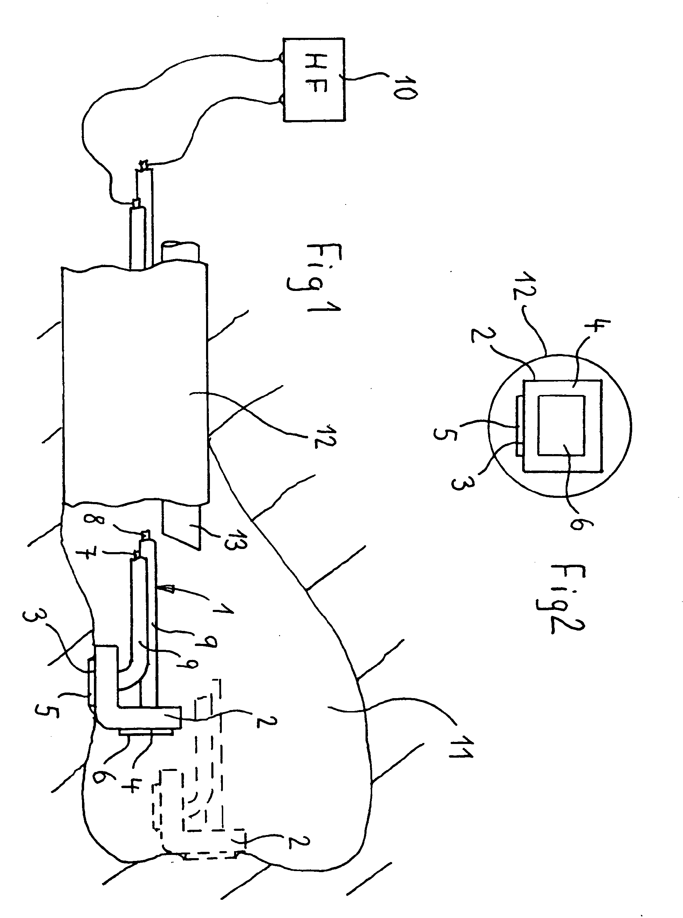

Auf den beiden Flächen 3, 4 sind je eine Elektrode 5, 6 flächig angeordnet. Die Elektroden 5, 6 sind im dargestellten Ausführungsfall als ebene Plattenelektroden ausgebildet. Ihre Größe ist jeweils kleiner als die Seitenfläche 3 bzw. die Stimfläche 4, so daß sie, wie insbesondere Figur 2 zeigt, in ihrer Fläche kleiner sind als die jeweilige Fläche des Isolatorkörpers 2 und somit allseitig von den Isolatorflächen 3, 4 überragt werden.

Die Elektroden 5, 6 sind durch den Isolatorkörper 2 hindurch mit drahtförmigen elektrischen Leitern 7, 8 kontaktiert, welche über ihre Länge hin mit elektrischer Isolierung 9 versehen sind. An den proximalen Enden sind die Leiter 7, 8, wie in Figur 1 dargestellt, an die beiden Pole eines HF-Generators 10 angeschlossen.

Figur 1 zeigt das typische Einsatzgebiet des dargestellten Instrumentes in einem Körperhohlraum 11, der nur schematisch dargestellt ist. Es kann sich dabei z.B. um die Blase handeln oder das Innere des Uterus. Bei typischer Anwendung bei der Prostataresektion kann es sich auch um den Hohlraum in einer Prostata handeln.

Von außen bis in den Körperhohlraum 11 hinein ist ein schematisch angedeutetes Endoskop verlegt mit einem Schaftrohr 12, durch dessen Lumen das dargestellte Instrument bis in den Hohlraum 11 vorschiebbar ist. Im Schaftrohr 12 ist ferner eine Optik 13 verlegt, unter deren Beobachtung mit dem dargestellten Instrument gearbeitet werden kann.

In Fig. 1 ist in einem Anwendungsbeispiel dargestellt, daß das Instrument mit der seitlichen Elektrode 5 in Gewebekontakt steht. Bei eingeschaltetem HF-Generator 10 fließt Strom von der seitlichen Elektrode 5 in das Körpergewebe und durch dessen benachbarte Oberfläche in den mit leitfähiger Flüssigkeit gefüllten Körperhohlraum 11 und durch die Flüssigkeit zur distalen Elektrode 6.

Wie aus Fig. 1 ersichtlich, könnte der Strom auch direkt zwischen den Elektroden fließen. Dann würde er nicht das Gewebe beeinflussen und nur nutzlose Verlustleistung produzieren. Wie Fig. 1 zeigt, wird der direkte Stromfluß durch die Flüssigkeit jedoch durch die Form des Isolatorkörpers 2 blockiert, der den direkten Stromfluß zwischen den Elektroden zu widerstandserhöhenden Umwegen zwingt. Ferner ergibt die großflächige Ausbildung der Seitenfläche 3 eine flächige Anlage dieser Seitenfläche auf dem Gewebe mit ihren die Elektrode 5 überragenden Randbereichen, wodurch direkte Strompfade durch die Flüssigkeit versperrt werden. Dadurch wird sehr wirkungsvoll der direkte Stromfluß zwischen den Elektroden blockiert.

In Fig. 1 ist gestrichelt angedeutet, daß das Instrument auch an der Stirnseite des Körperhohlraumes 11 mit der distalen Elektrode 6 in Gewebekontakt gebracht werden kann. Nun steht die seitliche Elektrode 5 in freiem Flüssigkeitskontakt. Der Strom fließt entsprechend, wie zuvor erläutert.

Wird das dargestellte Instrument um seine Achse gedreht, so daß die seitliche Elektrode 5 nach oben liegt, so kann auch am Dach des dargestellten Körperhohlraumes gearbeitet werden. Das dargestellte Instrument erlaubt also die Bearbeitung sämtlicher Oberflächenbereiche des Körperhohlraumes.

In der dargestellten Ausführungsform ist der Isolatorkörper 2 in abgewinkelter Grundform ausgebildet. Es kann jedoch auch eine geschlossene, z.B. im wesentlichen kubusförmige Grundform gewählt werden. Die Kanten des Isolators sind bevorzugt verrundet ausgebildet, um ungewollte Verletzungen des Gewebes zu vermeiden. Die Seitenfläche 3 und die Stirnfläche 4 des Isolatorkörpers 2 sind im Ausführungsbeispiel als ebene Flächen dargestellt. In einem Ausführungsbeispiel, das nicht Teil der vorliegenden Erfindung bildet, können sie auch leicht ballig ausgebildet sein, um beispielsweise in sehr kleinen Hohlräumen großflächigen Gewebekontakt zu ermöglichen.

Im dargestellten bevorzugten Ausführungsbeispiel sind großflächige Elektroden 5, 6 dargestellt. Die Elektroden können aber auch kleinflächig, z.B. knopfförmig in der Mitte der jeweiligen Flächen 3 und 4 des Isolatorkörpers 2 ausgebildet sein.

## Patentansprüche

1. Endoskopisches Instrument mit zwei an die beiden Pole eines HF-Generators (10) anschließbaren, am distalen Endbereich eines langgestreckten Einführungsteiles (1) angeordneten Elektroden (5, 6), zwischen denen ein Isolator (2) angeordnet ist, wobei eine der Elektroden (5) in bezug auf die Achse des Einführungsteiles (1) seitlich und die andere Elektrode (6) distal vom Isolator (2) angeordnet ist, **dadurch gekennzeichnet, daß** der Isolator als Körper (2) mit einer ebenen Seitenfläche (3) und einer senkrecht zur Achse des Einführungsteiles (1) stehenden, ebenen, distalen Stirnfläche (4) ausgebildet ist, wobei die Elektroden (5, 6) an diesen Flächen (3, 4) angeordnet sind.

2. Instrument nach Anspruch 1, **dadurch gekennzeichnet, daß** die Elektroden (5, 6) flächig auf den Flächen (3, 4) des Isolators (2) angeordnet sind.

3. Instrument nach Anspruch 2, **dadurch gekennzeichnet, daß** die Elektroden (5, 6) allseitig von den Flächen (3, 4) des Isolators überragt angeordnet sind.

## Claims

1. An endoscopic instrument with two electrodes (5, 6), which are connectable to the two poles of an HF generator (10) and are disposed on the distal end region of an elongate insertion portion (1) and disposed between which there is an insulator (2), wherein one of the electrodes (5) is disposed laterally with respect to the axis of the insertion portion (1) and the other electrode (6) is disposed distally of the insulator (2), **characterised in that** the insulator is in the form of a body (2) with a flat side surface (3) and a flat distal end surface (4) extending perpendicular to the axis of the insertion portion (1), the electrodes (5, 6) being arranged on these surfaces (3,4).

2. An instrument as claimed in Claim 1, **characterised in that** the electrodes (5, 6) are arranged flat on the surfaces (3, 4) of the insulator (2).

3. An instrument as claimed in Claim 2, **characterised in that** the surfaces (3, 4) of the insulator project beyond the electrodes (5, 6) on all sides.

## Revendications

1. Instrument endoscopique avec deux électrodes (5, 6) lesquelles peuvent être branchées sur les deux pôles d'un générateur H.F. (10) et sont agencées à l'extrémité distale d'une section d'introduction allongée (1) et entre lesquelles est placé un isolateur (2), l'une des électrodes (5) étant, relativement à l'axe de la section d'introduction (1) disposée latéralement et l'autre électrode (6) distalement par rapport à l'isolateur (2), **caractérisé en ce que**, l'isolateur est réalisé sous forme de corps (2) doté d'une face latérale (3) plane et d'une face frontale (4) plane distale orthogonale par rapport à l'axe de la section d'introduction (1), les électrodes (5, 6) étant disposées sur ces faces (3, 4).

2. Instrument selon la revendication 1, **caractérisé en ce que** les électrodes (5, 6) sont agencées en plan contre les faces (3, 4) de l'isolateur (2).

3. Instrument selon la revendication 2, **caractérisé en ce que** les électrodes (5, 6) sont agencées de façon à être dépassées de tous les côtés par les faces (3, 4) de l'isolateur.
